Europäisches Patentamt

European Patent Office

Office européen des brevets

(19)

(11) Publication number: **0 000 831**
**B1**

(12)           **EUROPEAN PATENT SPECIFICATION**

(45) Date of publication of patent specification: **16.03.83**

(51) Int. Cl.³: **A 61 M 25/00**

(21) Application number: **78300235.5**

(22) Date of filing: **03.08.78**

(54) A cannula assembly.

(30) Priority: **04.08.77 US 821848**

(43) Date of publication of application:
**21.02.79 Bulletin 79/4**

(45) Publication of the grant of the patent:
**16.03.83 Bulletin 83/11**

(84) Designated Contracting States:
**BE CH DE FR GB LU NL SE**

(56) References cited:
**BE - A - 851 299**
**DE - A - 2 645 520**
**LU - A - 77 252**
**US - A - 3 875 938**
**US - A - 4 016 879**

(73) Proprietor: **SORENSON RESEARCH CO. INC.**
**4387 Atherton Drive**
**Salt Lake City Utah 84107 (US)**

(72) Inventor: **Sorensen, James**
**2857 East 21st South**
**Salt Lake City, Utah 84109 (US)**
Inventor: **Reynolds, Gordon**
**4082 South Orchard Drive**
**Bountiful Utah 84010 (US)**
Inventor: **Ford, Dixon**
**1058 North Compton**
**Farmington Utah 84025 (US)**
Inventor: **Johnson, Robert**
**490 East 10600 South**
**Sandy, Utah 84070 (US)**

(74) Representative: **Walters, Frederick James et al,**
**Urquhart-Dykes & Lord 47 Marylebone Lane**
**London W1M 6DL (GB)**

A cannula assembly

Technical field

The present invention relates to a cannula assembly for medical use and more particularly to a double lumen cannula for independently conducting fluids into and out of a venipuncture site through two separate passageways.

Background art

Double lumen cannulae are well known in the art. The best known advantage offered by double lumen cannulae is the ability to separately withdraw blood from a blood vessel and inject blood back into the same blood vessel through a single venipuncture or fistula. The most common double lumen cannulae consist of a single tube with a horizontal division of the tube which places the lumen of the cannulae in immediate juxtaposition. Unfortunately, however, the construction of such double lumen cannulae is expensive, time-consuming and unreliable.

The least expensive and most reliable construction of the double lumen cannulae is in the form of concentric cannulae disposed telescopically one within the other. Particularly when using cannulae for single needle dialysis, it is preferred to have one cannula project substantially beyond the other. Attention is directed particularly to the common use of cannulae for single needle dialysis such as that disclosed and described in U.S. Patent 3,756,234. In single needle dialysis, it is highly desirable to withdraw blood from a patient, treat the blood with a hemodialyzer (artificial kidney) and return the blood to the patient through the same fistula in which the blood was withdrawn. Under such circumstances, it is desirable to return the blood a significant distance upstream from where the blood is aspirated so as to minimise the problem of admixing.

Belgian Patent Specification No. A851299 discloses a cannula assembly in which blood can be returned a small distance upstream from where the blood is aspirated by means of a telescopic arrangement of the cannulae. A needle is provided to puncture the skin of the patient but it will be appreciated in order for both cannulae to enter the blood vessel, not only is the needle used for penetration but also both cannulae. Unless there is a smooth contour between the penetrating member, in this case the needle, and the exterior cannula, the venipuncture is both difficult and painful. A relatively smooth exterior surface is described in German Patent Specification No. A 2645520 but the inner cannula which defines the penetrating member is supported by a sleeve. Generally, if telescoping cannulae present an exteriorly smooth surface for venipuncture, there is insufficient passageway for fluid to flow easily between them. The structure of a double lumen cannula lends itself admirably to the ability to aspirate blood from a downstream location and to return blood at an upstream location all through the same fistula. However, until this present invention, no suitable double lumen cannula enabling penetration into the bloodstream and for maintaining separate flow paths through a single fistula has been known.

Statement of invention and advantages

A first aspect of the present invention provides a cannula assembly comprising first and second cannulae that define separate flow paths, the second cannula extending, in a fluid transfer condition, concentrically through and forwardly of the first cannula, the assembly being characterised in that a needle is telescopically mounted therein to be displaceable between a position in a puncturing condition of the assembly in which the needle extends through the first cannula forwardly of both the first and second cannulae with the needle and first cannula defining a smooth exterior surface for venipuncture and a position in the fluid transfer condition of the assembly in which it is withdrawn rearwardly of the forward end of the first cannula and/or may be withdrawn rearwardly completely from the assembly, and further characterised in that the second cannula is withdrawn to a position rearward of the first cannula in the puncturing condition of the assembly and is displaceable forwardly in the fluid transfer, condition subsequent to withdrawal of the needle, the assembly being still further characterised in that the first and second cannulae and the needle are supported in a hollow body that is bifurcated and that is in communication with the first cannula, in that the second cannula is displaceable through one of the branches of the body which includes sealing means to control escape of fluid from said branch as the second cannula is displaced, and in that the needle is withdrawable from the other branch of the body from the puncturing condition with said other branch including a flexible sleeve which may be pinched closed when the needle is withdrawn therefrom to prevent escape of fluid through said branch.

A second aspect of the present invention provides a cannula assembly comprising first and second cannulae that define separate flow paths, the second cannula extending, in a fluid transfer condition, concentrically through and forwardly of the first cannula, and a needle telescopically mounted to be displaceable between a position in a puncturing condition of the assembly in which the needle extends through the first cannula forwardly of both the first and second cannulae and a position in the fluid transfer condition of the assembly in which it is withdrawn rearwardly of the forward end of the first cannula, the assembly being characterised in that the needle and first cannula define a smooth exterior surface for venipuncture, and in that the second cannula is mounted to extend through and forwardly of the first cannula during both the fluid transfer and the puncturing conditions of the assembly and the needle is telescopically slidable on the second cannula to extend between, and forwardly of, the first and second cannulae in the puncturing condition.

By the present invention, the smooth exterior contour between the needle and the first cannula facilitate venipuncture and yet when the needle is removed an adequate flow path may exist between the first and second cannulae.

Figures in the drawings

Various embodiments of cannula assemblies in accordance with the present invention will now be described by way of example only with reference to the accompanying drawings, in which:

Figure 1 is a perspective view of a first embodiment of the invention;

Figure 2 is a cross-section taken along lines 2—2 of Figure 1;

Figure 3 is a cross-sectional elevation of the embodiment of Figure 1 illustrating the stylet needle in a partially removed position and illustrating the interior cannula is partially advanced through the cannula hub or body;

Figure 4 is a cross-sectional elevation of the embodiment of Figure 1 illustrating the separate flow paths connected to appropriate extracorporeal blood circuitry;

Figure 5 is an enlarged cross-sectional elevation of a second embodiment similar to the embodiment of Figure 1 and illustrating a unidirectional flow control valve therein;

Figure 6 is a perspective illustration of a third embodiment having a reciprocating needle, the needle being illustrated in the forwardmost, initial position;

Figure 7 is a perspective illustration of the embodiment of Figure 6 with the needle in the retracted position; and

Figure 8 is a cross-section taken along lines 8—8 of Figure 7.

Detailed description of the drawings

Attention is now directed to the Figures wherein like parts are designated with like numerals throughout.

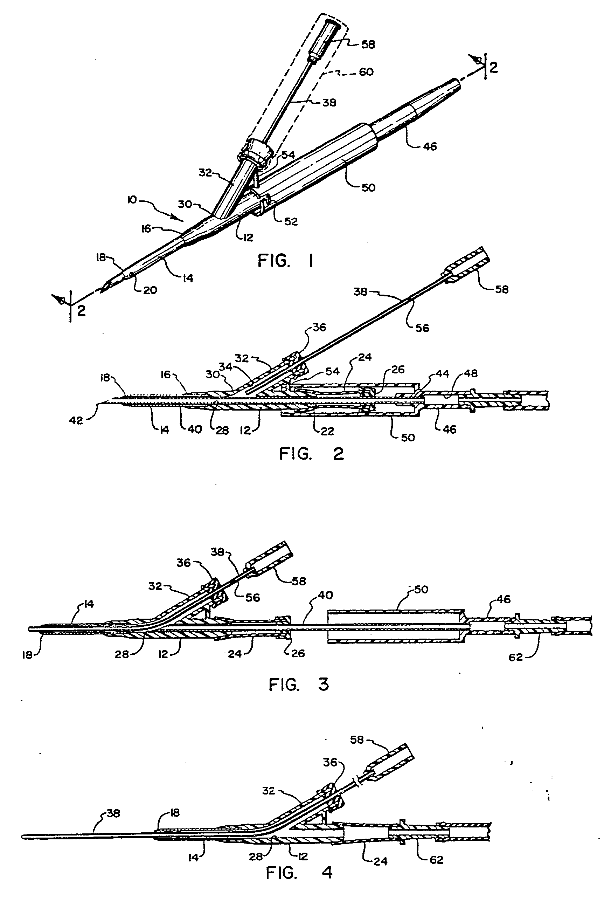

With particular reference to Figure 1, the cannula assembly generally designated 10 comprises a cannula body or hub 12 preferably formed of a rigid plastics material. An exterior hollow cannula 14 is mounted within the hub 12 at the leading end 16 thereof as shown best in Figure 2. The exterior cannula 14 projects forward of the hub 12 a discrete, predetermined distance and is tapered forwardly at the leading tip 18. If desired, apertures 20 may be formed in the exterior cannula 14 adjacent the leading tip 18 thereof. The hub 12 has a trailing end 22 which, in the illustrated embodiment, is secured to a flexible sleeve 24. The flexible sleeve 24 is provided with an end plug 26 preferably formed of neoprene or other suitable material which is self-sealing.

The hub 12 has an internal throughbore 28 which opens at the trailing end 22 of the hub and is in direct internal communication with the interior of cannula 14. The hub 12 is bifurcated at 30 so as to form a branch 32. Branch 32 has a bore 34 which communicates directly with the bore 28 of the hub 12. The branch 32 is rearwardly angularly related with respect to the hub 12 preferably at about 45 degrees or less, the bifurcation and angle facilitating displacement of an interior cannula 38 as hereinafter described. The branch 32 is provided with a sealing means such as neoprene self-sealing plug 36 through which the interior cannula 38 displaceably passes.

Referring again to Figure 2, an elongated stylet needle 40 is telescopically disposed through the entire length of the hub 12 and exterior cannula 14 and has an exterior diameter similar to the interior diameter of the cannula 14 thereby providing a smooth interchange surface. The sharpened tip 42 of the needle 40 projects beyond the exterior cannula 14 so as to permit facile venipuncture and safe introduction of the exterior cannula 14 into a blood vessel according to conventional techniques. The trailing end 44 of the needle 40 is rigidly mounted within a needle hub 46. Preferably, the needle hub 46 defines a hollow interior 48 which communicates directly with the interior of the hollow needle 40. Thus, upon successful venipuncture, the needle hub 46 will reflect a blood flashback indicating successful venipuncture. The needle is intended to pass immediately through the plug 26 in the position illustrated in Figure 2. However, because the sleeve 24 is flexible in configuration, and in order to give stability to the cannula assembly 10 during venipuncture, a cylindrical housing 50 is mounted upon the needle hub 46 and telescopically circumscribes the sleeve 24 and the trailing end 22 of the hub 12. As shown best in Figure 1, the housing 50 has a plurality of keyways 52 adapted to receive a suitable detent 54. The detent 54 in co-operation with the keyway 52 locks the housing 50 temporarily in place upon the hub 12.

After venipuncture, the housing 50 may be rotated slightly about the axis of the needle 40 and retracted rearwardly as shown in Figure 3 until the needle has been removed from the exterior cannula 14. While the needle is in the advanced position illustrated in Figure 2, the throughbore 28 is substantially obstructed. However, when the needle 40 is retracted to the position illustrated in Figure 3, at least that portion of the throughbore 28 which is forward of the intersection of bore 34 with bore 28 is unobstructed. When unobstructed, the interior cannula 38 may be advanced from its withdrawn position shown in Figure 2 through the hollow of the exterior cannula 14 by digital manipulation. The cannula 38 defines a hollow passageway 56 which communicates openly with the female coupling 58. The length of cannula 38 is selected to project beyond the leading end 18 of cannula 14 when the cannula 38 is fully advanced through the bore 34. When desired, blood may be communicated through the passageway 56 of the interior cannula 38 by connecting a blood tubing to the female coupling 58. Because the cannula 38 will come in contact with the patient's blood, it may be desirable to maintain the sterility of the cannula 38. Accordingly, a suitable flexible packaging material 60 as schematically illustrated in broken lines in Figure 1, could be used. Conventional packaging material 60 which permits advancement of a catheter through a hub is known in the art and no further comment thereon is deemed necessary.

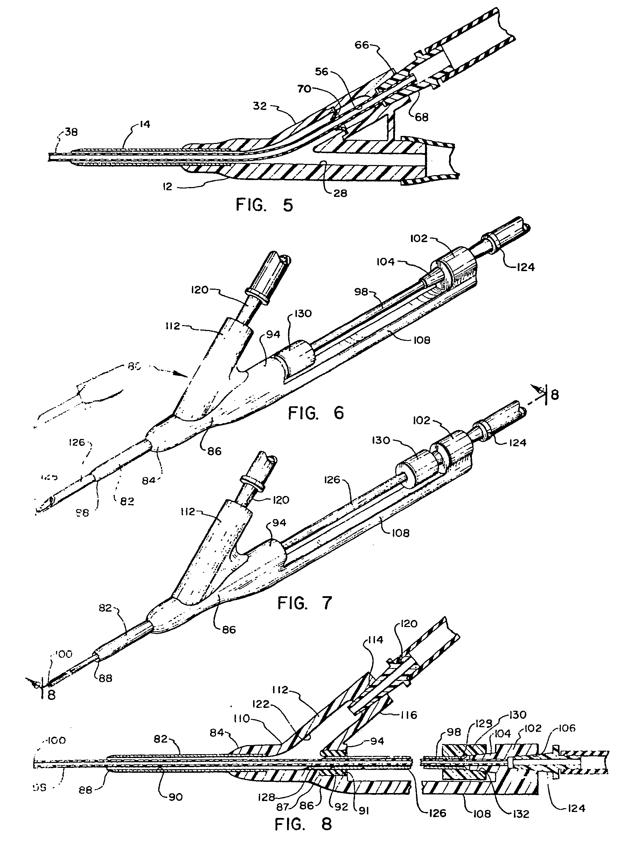

The method of using the cannula assembly embodiment of Figures 1 to 4 can best be understood by reference to Figures 3 and 4. With the needle in the fully advanced position illustrated in Figure 2, venipuncture is performed such that the exterior cannula 14 is inserted within a blood vessel to create a single fistula. Thereafter, the housing 50 is rotated slightly and the needle withdrawn to the position illustrated in Figure 3. Of course, if desired, the needle 40 may be completely withdrawn through the plug 26, the memory of the plug 26 preventing the blood from spilling out of the hub 12. However, it may also be desirable to leave the needle in the position illustrated in Figure 3. In any event, after the needle has been withdrawn from the exterior cannula 14, the interior cannula 38 may be advanced through the branch 32 until it projects a desired distance beyond the leading end 18 of the cannula 14. Thus, cannulae 14 and 38 are coextensive at least along the length of cannula 14 in the forward position of cannula 38. Notably, the external diameter of the cannula 38 is sufficiently less than the internal diameter of the cannula 14 that blood will flow easily therebetween. Accordingly, blood may be aspirated in the passageway between the cannula 14 and the cannula 38, through the bore 28, the hollow of the needle 40, the needle hub 46 and through extracorporeal blood circuitry (not shown) connected at 62. After the blood has traversed an extracorporeal blood circuit, it may then be returned through the coupling 58 and the passageway 56 within the interior cannula 38.

Note that blood may be withdrawn from and returned to the patient without creating an additional fistula and without otherwise modifying the structure of the cannula assembly 10. The withdrawal and return of blood may occur simultaneously or serially, as desired.

While the needle may be retained in the position illustrated in Figure 3, it has been found preferable to completely remove the needle 40 from the hub 12. Removal is easily accommodated by withdrawing the needle from the plug 26 manually squeezing the flexible sleeve 24 to prevent outflow of blood, removing plug 26 and thereafter inserting the coupling 62 of the extracorporeal blood circuit directly into the sleeve 24. In this configuration, the risk that the needle may damage the interior cannula 38 or otherwise interfere with the flow of blood is eliminated. As pointed out in Figure 4, in this presently preferred embodiment, the interior cannula 38 may be of any desirable length and will preferably project a substantial distance beyond the leading end 18 of the exterior cannula 14. Because the length of cannula 38 can be selected, any of a variety of distances between the leading tip 18 of cannula 14 and the leading tip of cannula 38 could be selected (see Figure 4).

Referring now to Figure 5, a modification of the embodiment of Figures 1—4 is illustrated. In Figure 5, a female Luer fitting 66 has been substituted for the rubber plug 36 as shown in Figure 2. The female Luer fitting receives a male coupling 68 in press-fit relation to permit return of blood from the extracorporeal blood circuit through the passageway 56 of the interior cannula 38.

In order to prevent blood within the throughbore 28 from escaping through the Luer fitting 66 as the cannula 38 is advanced, a unidirectional check valve 70 is provided in the branch 32. The check valve 70 is preferably made of a flexible rubber material through which the cannula 38 is easily passed in the forward direction. The valve 70 is a leaflet or duck-bill type valve.

Attention is now directed to another preferred cannula assembly embodiment generally designated 80 and illustrated in Figure 6—8. The cannula assembly 80 is intended to accomplish the same purpose as the assembly 10. More particularly, the assembly 80 comprises an exterior cannula 82 which is mounted at the forward end 84 of a cannula hub 86. Preferably, the exterior cannula 82 is forwardly tapered at 88 and defines an interior bore 90 (see Figure 8). The bore 90 of the cannula 82 communicates directly with a recess 91 in the hub 86. The recess 91 opens at the trailing end 94 of the hub 86. Rubber sleeve 87 is nested within recess 91 and defines a throughbore 92 which tightly circumscribes needle 126 to form an air-tight seal as will hereinafter be more fully described.

An interior catheter or cannula 98 traverses the entire throughbore 92 and the bore 90 of exterior cannula 82 in fixed manner. The leading end 100 of the cannula 98 projects substantially beyond the leading end 88 of cannula 82 as shown in both Figures 7 and 8 and a fluid flow path is provided between the two cannulae. The trailing end of cannula 98 is mounted within coupling member 102, the coupling member 102 having a male Luer fitting 104 which is in direct alignment with the throughbore 92. The interior cannula 98 is hollow and opens directly into the female Luer coupling 106. The coupling member 102 is maintained in direct alignment and fixed spacial relationship with the hub 86 by a strut 108. It can be seen, therefore, that there is a discrete passageway from the leading tip 100 of the cannula 98 through the entire length of the cannula 98 to the coupling member 102.

The cannula hub 86 is bifurcated at 110 to form a rearwardly angled branch 112. The branch 112 has a female Luer fitting 114 at its trailing end 116 into which a suitable coupling 120 from a conventional extracorporeal blood circuit is press-fit. The branch 112 has a hollow interior 122 which communicates directly with the bore 90 of exterior cannula 82. Accordingly, a discrete passageway exists through the bore 90 and hollow 122 to the coupling 120. In addition as previously described, a separate and discrete passageway exists through the hollow of interior cannula 98 to the trailing end of coupling member 102 and to a male coupling 124 press-fit therein.

In order to facilitate introduction of the coextensive cannulae 82 and 98, a venipuncture needle 126 is provided. The venipuncture needle 126 has a sharp beveled end 128 which, in the initial position illustrated in Figure 6, projects through the interior of cannula 82 and telescopically around and beyond cannula 98. With reference to Figure 8, it can be appreciated that the needle 126 is reciprocably displaceable within the bore 92 of the sleeve 87. The sleeve 87 forms an air-tight seal with needle 126 so that negative pressure within the hub 86 and branch 112 will not draw air around the needle 126 into the blood within the hub. The trailing end 129 of the needle 126 is firmly mounted to a shuttle 130, the shuttle having a rearwardly facing female fitting 132 which is selectively press-fit upon the male fitting 104 of coupling member 102 as illustrated in Figure 8. Clearly, the location of the male fitting 104 and female fitting 132 could be reversed without adverse consequence. This press-fit coupling minimises blood leakage around the needle 126 after venipuncture and forms an air seal for preventing air from entering the hub 86 when the interior of hub 86 is subjected to negative internal pressure.

The method of using the embodiment of Figures 6—8 is apparent from the drawing. Initially, the shuttle 130 is advanced forwardly until the needle 126 projects beyond the leading end 88 of the cannula 82 and substantially circumscribes and confines the interior cannula 98. By restraining the shuttle 130 in place with the fingers, the venipuncture can easily be made. It is observed, however, that the needle 126 will substantially fill the passageway in the throughbore 90 between the cannula 82 and the cannula 98 thereby providing a smooth surface between itself and the cannula 82. Accordingly, blood will not flow through the bore 90 and hollow 122 in the branch 112 while the needle is in the forward position illustrated in Figure 6.

After venipuncture has been successfully accomplished and the fistula established, the shuttle 130 may be manually displaced rearwardly and telescopically over the exposed portion of the interior cannula 98 until the shuttle 130 mates with the coupling member 102. In this mode, blood will freely flow between the cannulae 82 and 98, through the branch 112 and the extracorporeal blood circuit connected at 120. At the same time, blood may also flow through the hollow of the interior cannula 98 and through the coupling 124 to the extracorporeal blood circuit.

The length of the needle 126 and the strut 108 are selected so that when the shuttle 130 is in the rearmost position illustrated in Figures 7 and 8, the sharpened leading end 128 of the needle 126 will remain supported by the cannula hub 86 but be fully retracted out of the pathway 122 so as to avoid interfering with blood flow.

Industrial exploitation

Clearly, in the embodiments illustrated herein a structure has been described which facilitates successful cannulation of a blood vessel and, after cannulation, the displacement and/or removal of the sharpened needle to permit essentially simultaneous aspiration of blood from the patient and delivery of blood back to the patient through two discrete flow paths in the assembly.

The invention may be embodied in other specific forms without departing from its spirit or essential characteristics. The described embodiments are to be considered in all respect only as illustrative and not restrictive and the scope of the invention is, therefore, indicated by the appended claims rather than by the foregoing description. All changes which come within the meaning and range of equivalency of the claims are to be embraced within their scope.

**Claims**

1. A cannula assembly (10) comprising first and second cannulae (14, 38) that define separate flow paths, the second cannula (38) extending, in a fluid transfer condition, concentrically through and forwardly of the first cannula (14), the assembly (10) being characterised in that a needle (40) is telescopically mounted therein to be displaceable between a position in a puncturing condition of the assembly (10) in which the needle (40) extends through the first cannula (14) forwardly of both the first and second cannulae (14, 38) with the needle (40) and first cannula (14) defining a smooth exterior surface for venipuncture and a position in the fluid transfer condition of the assembly in which it is withdrawn rearwardly of the forward end (18) of the first cannula (14) and/or may be withdrawn rearwardly completely from the assembly (10), and further characterised in that the second cannula (38) is withdrawn to a position rearward of the first cannula (14) in the puncturing condition of the assembly (10) and is displaceable forwardly in the fluid transfer condition subsequent to withdrawal of the needle (40), the assembly (10) being still further characterised in that the first and second cannulae (14, 38) and the needle (40) are supported in a hollow body (12) that is bifurcated and that is in communication with the first cannula (14), in that the second cannula (38) is displaceable through one of the branches (32) of the body (12) which includes sealing means (36, 70) to control escape of fluid from said branch (32) as the second cannula (38) is displaced, and in that the needle (40) is withdrawable from the other branch (22) of the body (12) from the puncturing condition with said other branch (22) including a flexible sleeve (24) which may be pinched closed when the needle (40) is withdrawn therefrom to prevent escape of fluid through said branch (22).

2. A cannula assembly according to claim 1 characterised in that the sealing means comprises a unidirectional check valve (70) located within the hollow body (12) and through which the second cannula (38) is displaceable.

3. A cannula assembly according to claim 1 or claim 2 characterised in that the needle (40) is further supported by means of an elongate housing (50) which in the puncturing condition of the assembly (10) extends over the flexible sleeve (24) and releasably engages with the body (12).

4. A cannula assembly (80) comprising first and second cannulae (82, 98) that define separate flow paths, the second cannula (98) extending, in a fluid transfer condition, concentrically through and forwardly of the first cannula (82), and a needle (126) telescopically mounted to be displaceable between a position in a puncturing condition of the assembly (80) in which the needle (126) extends through the first cannula (82) forwardly of both the first and second cannulae (82, 98) and a position in the fluid transfer condition of the assembly (80) in which it is withdrawn rearwardly of the forward end (88) of the first cannula (82), the assembly (80) being characterised in that the needle (126) and first cannula (82) define a smooth exterior surface for venipuncture, and in that the second cannula (98) is mounted to extend through and forwardly of the first cannula (82) during both the fluid transfer and the puncturing conditions of the assembly (80) and the needle (126) is telescopically slidable on the second cannula (98) to extend between, and forwardly, of, the first and second cannulae (82, 98) in the puncturing condition.

5. A cannula assembly according to claim 4 characterised in that the first and second cannulae (82, 98) and the needle (126) are supported in a hollow body (86) that communicates with the first cannula (82), and in that the withdrawn position of the needle (126) is determined by a stop (102) secured to the body (86) at a spaced location rearwardly thereof, the rearward end (129) of the needle (126) and the stop (102) having complementary coupling portions (132 104) which engage in the fluid transfer condition to prevent escape of the fluid from the hollow of the needle (126), and further in that the stop (102) is in communication with the second cannula (98) which extends through the body (86).

6. A cannula assembly according to claim 5 characterised in that an air-seal (87) is provided between the needle (126) and the body (86).

**Patentansprüche**

1. Kanülenaufbau mit einer ersten und zweiten Kanüle (14, 38; 82, 98), welche separate Durchströmkanäle bilden, wobei sich die zweite Kanüle (38; 98) in einer fluidübertragenden Stellung konzentrisch durch und nach vorne aus der ersten Kanüle (14; 82) erstreckt, dadurch gekennzeichnet, daß im Kanülenaufbau (10; 80) eine Nadel (40; 126) teleskopartig und zwischen einer Position in einer Punktierstellung des Kanülenaufbaus, in welcher sich die Nadel durch die erste Kanüle (14; 82) nach vorne aus der ersten und zweiten Kanüle (14, 38; 82, 98) erstreckt, und einer Position in der fluidübertragenden Stellung des Kanülenaufbaus verschiebbar angeordnet ist, in welcher die Nadel nach hinten aus dem vorderen Ende der ersten Kanüle (14; 38) zurückgezogen ist, und daß die Nadel (40; 126) und die erste Kanüle (14; 38) eine glatte Außenfläche zur Venenpunktion bilden.

2. Aufbau nach Patentanspruch 1, dadurch gekennzeichnet, daß die zweite Kanüle (38; 98) in der Punktierstellung des Kanülenaufbaus (10; 80) in eine Position hinter der ersten Kanüle (14; 28) zurückgezogen und in der fluidübertragenden Stellung nach dem Zurückziehen der Nadel (40; 126) nach vorne verschoben ist.

3. Aufbau nach Patentanspruch 2, dadurch gekennzeichnet, daß die erste und zweite Kanüle (14, 38; 82, 98) und die Nadel (40; 126) in einem (bei 30; 110) gegabelten und mit der ersten Kanüle (14; 82) in Verbindung stehenden Hohlkörper (12; 86) gelagert sind, und daß die zweite Kanüle (38; 98) durch einen der Schenkel (32; 112) des Hohlkörpers verschiebbar ist, welcher eine Dichteinrichtung zur Regulierung des Fluidabflusses von Schenkel bei verschobener zweiter Kanüle besitzt.

4. Aufbau nach Patentanspruch 3, dadurch gekennzeichnet, daß die Dichteinrichtung ein in einer Richtung wirkendes Absperrventil (70) besitzt, welches innerhalb des Hohlkörpers (12) angeordnet ist und durch welches die zweite Kanüle (38) verschiebbar ist.

5. Aufbau nach einem der Patentansprüche 3 und 4, dadurch gekennzeichnet, daß die Nadel (40; 126) in oder aus dem anderen Schenkel des Hohlkörpers (12; 86) aus der Punktierstellung des Kanülenaufbaus (10; 80) zurückziehbar ist.

6. Aufbau nach Patentanspruch 5, dadurch gekennzeichnet, daß der andere Schenkel eine flexible Hülse (24) besitzt, welche nach Zurückziehen der Nadel (40) von der Hülse durch Zusammenklemmen verschließbar ist, um ein Ausfließen von Fluid durch den Schenkel zu verhindern.

7. Aufbau nach Patentanspruch 6, dadurch gekennzeichnet, daß die Nadel (40) ferner mittels eines langgestreckten Gehäuses (50) gelagert ist, welches in der Punktierstellung des Kanülenaufbaus (10) sich über die flexible Hülse (24) erstreckt und ausrückbar mit dem Körper (12) zusammenwirkt.

8. Aufbau nach Patentanspruch 1, dadurch gekennzeichnet, daß die zweite Kanüle (38; 98) so angeordnet ist, daß sie sich sowohl während der fluidübertragenden Stellung und der Punktierstellung des Kanülenaufbaus (10; 80) durch und aus der ersten Kanüle (14; 82) erstreckt, und daß die Nadel (40; 126) an der zweiten Kanüle (38; 98) teleskopartig verschiebbar ist, so daß sie sich in der Punktierstellung zwischen der ersten und zweiten Kanüle und nach vorne aus den Kanülen (14, 38; 82, 98) erstreckt.

9. Aufbau nach Patentanspruch 8, dadurch gekennzeichnet, daß die erste und zweite Kanüle (14, 38; 82, 98) und die Nadel (40; 126) in einem Hohlkörper (12; 86) gelagert sind, der mit der ersten Kanüle (14; 82) in Verbindung steht, und daß die zurückgezogene Stellung der Nadel (40; 126) durch einen Anschlag bestimmt ist, welcher mit Abstand in einer hinteren Lage am Körper befestigt ist, wobei das rückwärtige Ende der Nadel und der Anschlag komplementäre Kupplungsabschnitte besitzen, welche in der fluidübertragenden Stellung zusammenwirken, um ein Ausfließen von Fluid aus dem Hohlraum der Nadel (40; 126) zu verhindern, und daß der Kupplungsabschnitt des Anschlags mit der zweiten Kanüle (38; 98) in Verbindung steht, welche sich durch den Körper (12; 86) erstreckt.

10. Aufbau nach Patentanspruch 7, dadurch gekennzeichnet, daß zwischen der Nadel (126) und dem Körper (86) eine luftdichte Abdichtung (87) vorgesehen ist.

## Revendications

1. Ensemble de canules comprenant une première et une seconde canules qui définissent des voies d'écoulement séparées, la seconde canule s'étendant, dans une condition de transfert de sang concentriquement à travers et vers l'avant de la première canule, ensemble caractérisé en ce qu'une aiguille est montée télescopiquement à l'intérieur, pour être déplaçable entre une position dans une condition de l'ensemble propre à une piqûre, dans laquelle l'aiguille s'étend vers l'avant à travers la première canule et, à la fois, la première et la seconde canules, et une position dans la condition de transfert de fluide de l'ensemble, dans laquelle elle est rétractée vers l'arrière de l'extrémité antérieure de la première canule, ensemble caractérisé en outre en ce que l'aiguille et la première canule définissent ensemble une surface extérieure lisse pour la piqûre intraveineuse.

2. Ensemble de canules suivant la revendication 1, caractérisé en ce que la seconde canule est retirée vers une position en arrière de la première canule dans la position de l'ensemble propre à la piqûre, et est déplaçable vers l'avant dans la condition de transfert de fluide, après retrait de l'aiguille.

3. Ensemble de canules suivant la revendication 2, caractérisé en ce que la première et la seconde canules et l'aiguille sont supportées dans un corps creux qui est bifurqué et qui est en communication avec la première canule, la seconde canule étant déplaçable à travers l'une des branches du corps qui comporte des moyens d'étanchéité pour s'opposer à un échappement du fluide hors de cette branche lorsque la seconde canule est déplacée.

4. Ensemble de canules suivant la revendication 3, caractérisé en ce que les moyens d'étanchéité comprennent une soupape d'arrêt unidirectionnelle disposée à l'intérieur du corps creux et à travers laquelle la seconde canule est déplaçable.

5. Ensemble de canules suivant l'une quelconque des revendications 3 et 4, caractérisé en ce que l'aiguille peut être retirée à l'intérieur ou hors de l'autre branche du corps, à partir de la condition de piqûre de l'ensemble.

6. Ensemble de canules suivant la revendication 5, caractérisé en ce que l'autre branche comprend un manchon flexible qui peut être fermé par pincement lorsque l'aiguille en est retirée, de manière à s'opposer à un échappement du fluide à travers cette branche.

7. Ensemble de canules suivant la revendication 6, caractérisé en ce que l'aiguille est, en outre, supportée au moyen d'un carter d'enveloppe allongé, lequel dans la condition de piqûre de l'ensemble, s'étend par dessus le manchon flexible et est engagé de manière libérable avec le corps.

8. Ensemble de canules suivant la revendication 1, caractérisé en ce que la seconde canule est montée de manière à s'étendre à travers et en avant de la première canule pendant les conditions de piqûre et de transfert de fluide de l'ensemble, et l'aiguille est coulissante télescopiquement sur la seconde canule pour s'étendre entre la première et la seconde canules et en avant de celles-ci, dans la condition de piqûre.

9. Ensemble de canules suivant la revendication 8, caractérisé en ce que la première et la seconde canules et l'aiguille sont supportées dans un corps creux qui communique avec la première canule, la position de retrait de l'aiguille étant déterminée par une butée d'arrêt fixée sur le corps à un emplacement espacé vers l'arrière de celui-ci, l'extrémité tournée vers l'arrière de l'aiguille et la butée ayant des portions d'accouplement complémentaires qui s'engagent entre elles, dans la condition de transfert de fluide, pour éviter l'échappement de fluide hors de l'espace creux de l'aiguille, la portion d'accouplement de la butée étant en communication avec la seconde canule qui s'étend à travers le corps.

10. Ensemble de canule suivant la revendication 7, caractérisé en ce que un joint étanche à l'air est prévu entre l'aiguille et le corps.

FIG. 1

FIG. 2

FIG. 3

FIG. 4

FIG. 5

FIG. 6

FIG. 7

FIG. 8

2